# FASCICULE DE BREVET EUROPEEN

(11) **EP 0 829 485 B1**
(45) Date de publication et mention de la délivrance du brevet: **20.06.2001**
(21) Numéro de dépôt: 97402118.0
(22) Date de dépôt: 12.09.1997
(51) Int. Cl.: C07H 3/02

(54) **Procédé de fabrication de D-érythrose**
Verfahren zur Herstellung von D-Erythrose
Method of manufacturing D-erythrose

(30) Priorité: 16.09.1996 FR 9611255
(43) Date de publication de la demande: 18.03.1998
(73) Titulaire: ROQUETTE FRERES, 62136 Lestrem (FR)
(72) Inventeur: Fleche, Guy, 59190 Hazebrouck (FR); Tamion, Rodolphe, 62157 Allouagne (FR)
(74) Mandataire: Boulinguiez, Didier

(56) Documents cités:
- EP-A- 0 716 067
- US-A- 2 986 495
- RUFF: "d-Erythrose", BERICHTE, , 1899, Vol. 32, no. , pages 3672 à 3681

## Description

La présente invention a pour objet un procédé de fabrication de D-érythrose.

Plus précisément, la présente invention a pour objet un procédé de fabrication de D-érythrose à partir d'acide gluconique, catalysé par les ions d'un métal choisi dans le groupe constitué du cobalt, du nickel et du ruthénium, et se déroulant en phase aqueuse.

On connaît déjà des procédés de fabrication de D-érythrose.

Parmi ceux-ci, on peut citer en tout premier lieu celui mis au point par RUFF (Ber. 32, 3674 (1899) ; 33, 1799 (1900)) et qui consiste à oxyder le D-arabinonate de calcium en présence d'eau oxygénée. Un tel procédé présente comme inconvénient majeur d'utiliser comme matière première l'acide arabinonique qui n'est pas un produit courant du commerce.

D'autres procédés de fabrication du D-érythrose ont suivi, comme l'oxydation du D-glucose en présence de tétraacétate de plomb, connu sous le nom de méthode de PERLIN (Perlin A.S., Methods Carbohydr. Chem., 1962, 1, 64), ou l'hydrolyse acide du 2,4-0-éthylidène-D-érythrose obtenu par l'oxydation au périodate du 4,6-O-éthylidène-D-glucose (Schaffer R., J. Am. Chem. Soc. 81 (1959) 2838 ; Barker R. and Mac Donald D.L.,J. A. Chem. Soc. 82 (1960) 2301).

Le D-érythrose, en tant que tel, ne présente pas un grand intérêt, mais serait surtout un intermédiaire de synthèse fort important s'il advenait que l'on puisse le produire en grande quantité et à faible coût.

En effet, une simple étape complémentaire d'hydrogénation du D-érythrose permet d'obtenir facilement l'érythritol qui est un polyol pouvant être employé dans de multiples applications alimentaires, et notamment en tant que substitut non cariogène et hypocalorique du saccharose.

De ce fait, la présente invention a également pour objet un procédé de fabrication d'érythritol à partir de D-érythrose obtenu conformément au procédé de l'invention.

Bien que répandu dans la nature et assimilé de tout temps par l'homme à travers son alimentation, l'érythritol a longtemps été ignoré par l'industrie alimentaire, en raison des difficultés rencontrées à l'obtenir de façon rentable.

Dans l'industrie pharmaceutique, l'érythritol permet d'accéder par oxydation au L-érythrulose, molécule qui possède une fonctionnalité intéressante lui conférant la possibilité d'être utilisée pour la synthèse de composés biologiquement actifs.

Les études ayant porté sur les procédés de fabrication de l'érythritol se sont globalement partagées entre deux grandes voies : la synthèse chimique et la biosynthèse par voie fermentaire.

Aucune des techniques connues en synthèse chimique comme la réduction du méso-tartrate, l'oxydo-réduction du D-éthylidène 4,6 D-glucose et l'hydrogénation d'hydrolysats d'amidon dialdéhyde (T. Dola et T. Sasaki, Bio-Industry, (1988), 5, (9), 32) n'a cependant pu atteindre une véritable dimension industrielle.

Bien que nettement plus nombreux qu'en synthèse chimique, les travaux menés sur les techniques de fermentation s'intéressent dans leur grande majorité à la production d'érythritol à titre de constituant secondaire.

Ces travaux se sont appliqués à la production d'érythritol par les levures *Debaryomyces* (US-A 2.986.495), *Pichia* (US-A 2.986.495), *Candida* (US-A 3.756.917), *Moniliella* (Antonie van Leeuwenhoek, 37 (1971) 107-118), et *Aureobasidium* (JP-A 61/31.091).

Les résultats fournis à ce jour par les études sur la fermentation de l'érythritol mettent néanmoins en exergue un certain nombre d'inconvénients qui, comme le moussage en cours de fermentation, la vitesse de fermentation, l'importance des sous-produits et surtout le mauvais rendement en compromettent encore les possibilités d'industrialisation.

Il existait donc un besoin de mettre au point un procédé performant de fabrication de D-érythrose (et donc d'érythritol par hydrogénation du D-érythrose ainsi obtenu) ne présentant pas les limites et/ou les inconvénients de l'art antérieur.

C'est en travaillant sur ce thème de recherche que la Demanderesse a mis au point un nouveau procédé de fabrication du D-érythrose par voie chimique à partir d'acide gluconique ou de ses sels. Le procédé conforme à l'invention reprend le principe de la méthode exposée par RUFF il y a près d'un siècle.

Cette méthode permet de passer, de façon générale, d'un acide aldonique comportant n carbones à un aldose comportant (n-1) carbones grâce à l'action combinée d'ions ferriques et d'eau oxygénée. Cependant, les rendements en aldose sont très modestes.

Ainsi, la conversion de l'acide gluconique en D-arabinose est réalisée selon cette méthode.

Quelques améliorations ont, par la suite, été apportées par R.C. Hockett et C.S. Hudson (J. Amer. Chem. Soc. 56, 1632-1633, (1934) et ibid. 72, 4546, (1950)) et par le document US-A 3.755.294. Des rendements de 60 % d'arabinose en partant d'acide gluconique y sont décrits. Un progrès a été accompli par V. Bilik (CZ - 232647, (1983)) en utilisant les ions cuivriques (Cu (II)) comme catalyseurs. Des rendements de l'ordre de 70 % sont atteints après une purification laborieuse.

Des résultats identiques ont été obtenus récemment avec un mélange d'ions ferriques et ferreux comme catalyseurs (CZ - 279002, (1994)).

Enfin, dans des conditions particulières, le document EP-A 0.716.067 rapporte des rendements de 78 % en certains aldoses.

Durant une investigation large de la réaction de RUFF, la Demanderesse a découvert que les sels de cobalt, de nickel, ainsi que ceux de ruthénium catalysaient la réaction de l'acide gluconique avec l'eau oxygénée pour donner, de façon surprenante, le D-érythrose et non, comme on pouvait s'y attendre, le D-arabinose. Il y a donc perte de deux atomes de carbone par rapport à l'acide aldonique de départ.

Ainsi selon l'invention, le procédé de fabrication de D-érythrose est caractérisé par le fait qu'une solution aqueuse d'un sel de l'acide gluconique est mise au contact de peroxyde d'hydrogène en présence d'un sel d'un métal choisi dans le groupe constitué du cobalt, du nickel et du ruthénium.

Un premier avantage d'un tel procédé par rapport aux procédés de fermentation de l'art antérieur est évidemment d'éviter toutes les contraintes et problèmes liés aux techniques de fermentation tels que mentionnés ci-dessus.

Un deuxième avantage du procédé conforme à l'invention réside dans le fait qu'il est extrêmement facile à mettre en oeuvre puisque tant la matière première que les réactifs sont facilement accessibles.

Un troisième avantage du procédé conforme à l'invention est que le D-érythrose est obtenu avec un très bon rendement proche de la stoechiométrie.

Un autre avantage du procédé conforme à l'invention est qu'il trouve sa place aisément dans l'industrie, notamment alimentaire, du fait qu'il utilise de l'eau comme solvant, avantage indéniable tant au plan de la toxicité qu'au plan de la sécurité.

Le procédé de l'invention met en oeuvre un sel de l'acide gluconique.

Dans la présente invention, on entend par sel de l'acide gluconique, l'acide gluconique sous forme libre, sous forme lactonisée ou sous forme de mélange de ces deux formes, sous forme de sels ou sous forme d'esters. Ainsi, par exemple le gluconate de calcium, le gluconate de sodium ou la glucono-δ-lactone conviennent parfaitement.

L'acide gluconique est obtenu de manière connue par oxydation de glucose. Cette étape d'oxydation peut être conduite soit par voie chimique, soit par voie microbiologique.

La voie chimique préférée dans le cadre de l'invention consiste à oxyder le glucose à l'aide d'air ou d'oxygène en milieu alcalin et à l'aide de catalyseurs au palladium.

Un procédé particulièrement préféré est celui qui a été décrit dans le brevet américain US-A 4.845.208, dont la Demanderesse est cessionnaire, qui consiste à utiliser comme catalyseur d'oxydation, du palladium fixé sur charbon actif et dopé au bismuth.

On peut également envisager d'oxyder le glucose par voie électrolytique ou à l'aide d'hypobromite. On peut encore oxyder le glucose par voie microbiologique à l'aide de *Gluconobacter* ou *d'Aspergillus.*

De préférence le procédé de l'invention est mis en oeuvre, dans l'eau, avec une teneur en matière sèche en sel de l'acide gluconique comprise entre 1 et 60 %, préférentiellement entre 5 et 50 % et plus particulièrement entre 10 et 30 %.

Les contraintes de matière sèche inférieure sont imposées pour des raisons évidentes d'économie d'évaporation de l'eau et de réduction de la taille des réacteurs.

Les contraintes de matière sèche supérieure sont essentiellement imposées par des problèmes de solubilité ou de viscosité du milieu réactionnel.

Dans la présente description, tous les pourcentages sont exprimés par rapport à l'acide gluconique (exemple : 50 mol% signifie 50 moles de X pour 100 moles d'acide gluconique, et 50 % signifie 50 g de X pour 100 g d'acide gluconique au départ).

Dans le procédé conforme à l'invention, le catalyseur est constitué par des ions d'un métal choisi dans le groupe constitué du cobalt, du nickel et du ruthénium qui peuvent être amenés sous forme d'un sel quelconque de cobalt, de nickel ou de ruthénium divalent ou trivalent. Avantageusement, on préfère les sels de cobalt : acétate, acétylacétonate, halogénures, nitrate, sulfate, de cobalt par exemple conviennent parfaitement.

Une quantité de catalyseur (sel de cobalt, de nickel ou de ruthénium) comprise entre 0,001 et 50 %, préférentiellement entre 0,002 et 20 % et plus particulièrement entre 0,005 et 5 % par rapport au sel de l'acide gluconique mis en oeuvre donne de bons résultats dans le procédé conforme à l'invention, tant en ce qui concerne le rendement que la pureté du D-érythrose obtenu.

Au mélange de sel d'acide gluconique, de catalyseur et d'eau ainsi réalisé, on ajoute alors lentement sous agitation, le peroxyde d'hydrogène, de préférence sous forme d'eau oxygénée d'une richesse de 30 %, à raison de 1 à 500 mol%, préférentiellement de 50 à 400 mol% et plus particulièrement de 100 à 300 mol% par rapport au sel de l'acide gluconique mis en oeuvre.

Il est possible d'utiliser du peroxyde d'hydrogène sous forme d'eau oxygénée d'une richesse supérieure à 30 %, notamment par exemple jusqu'à 70 %.

L'addition de l'eau oxygénée est effectuée à une vitesse d'introduction telle que la température du milieu réactionnel ne s'élève pas, de préférence, au-delà de 50°C et plus particulièrement au-delà de 35°C. Ainsi, la vitesse d'introduction de l'eau oxygénée se situe généralement entre 30 minutes et deux heures.

De préférence, le procédé de l'invention est mis en oeuvre à une température comprise entre 0 et 100°C et préférentiellement entre 10 et 50°C.

Des températures inférieures amènent des temps de réaction trop longs et des températures supérieures, outre qu'elles nécessiteraient l'emploi de réacteurs résistants à la pression, conduiraient à une dégradation des produits de la réaction.

Les températures de 20 à 40°C sont donc particulièrement préférées dans le procédé de l'invention.

De préférence encore, le procédé de l'invention est mis en oeuvre à un pH compris entre 2 et 12, préférentiellement compris entre 5 et 8 et plus particulièrement compris entre 6 et 7.

Le D-érythrose obtenu conformément au procédé de l'invention, sous sa forme brute, peut alors être facilement hydrogéné catalytiquement.

L'hydrogénation d'un tel sucre s'effectue conformément aux règles de l'art qui conduisent par exemple à la production de sorbitol à partir du glucose.

On peut utiliser pour cette étape aussi bien des catalyseurs à base de ruthénium que des catalyseurs au nickel de Raney.

On préfère cependant utiliser des catalyseurs au nickel de Raney qui sont moins onéreux.

Dans la pratique, on utilise de 1 à 10 % en poids de catalyseur par rapport à la matière sèche de sucre soumis à l'hydrogénation. L'hydrogénation s'effectue de préférence sur des sirops dont la matière sèche est comprise entre 15 et 50 %, dans la pratique voisine de 30 à 45 %, sous une pression d'hydrogène comprise entre 20 et 200 bars. Elle peut être effectuée de manière continue ou discontinue.

Lorsque l'on opère de manière discontinue, la pression d'hydrogène utilisée est généralement comprise entre 30 et 60 bars et la température à laquelle se déroule l'hydrogénation est comprise entre 100 et 150°C. On veille aussi à maintenir le pH du milieu d'hydrogénation par l'addition de soude ou de carbonate de soude par exemple, mais sans dépasser un pH de 9,0. Cette manière de faire permet d'éviter l'apparition de produits de cracking ou d'isomérisation.

On arrête la réaction lorsque la teneur du milieu réactionnel en sucres réducteurs est devenue inférieure à 1 %, de préférence encore inférieure à 0,5 % et plus particulièrement inférieure à 0,1 %.

Après refroidissement du milieu réactionnel, on élimine le catalyseur par filtration et on déminéralise le D-érythritol ainsi obtenu sur des résines cationiques et anioniques.

A ce stade, les sirops contiennent au moins 90 % de D-érythritol et il est aisé d'en purifier celui-ci par cristallisation après concentration et refroidissement des solutions.

L'invention sera mieux comprise au moyen des exemples qui suivent et qui ont pour seul but de mieux illustrer l'invention sans vouloir la réduire aux modes de réalisations expressément décrits et au seul gluconate de calcium mis en oeuvre.

Dans les exemples qui suivent, tous les résultats sont exprimés en pourcentage molaire.

### EXEMPLE 1 :

Le gluconate de calcium monohydrate (115,7 g, 0,255 mole), le chlorure de cobalt hexahydrate (0,58 g, 2,4 mmoles) et de l'eau (1000 ml) sont introduits dans un réacteur à double enveloppe.

Le mélange est porté à une température de 30°C et à un pH de 6,5 par addition de soude 2N.

L'eau oxygénée (130 ml, 1,28 moles) est introduite en 70 minutes en maintenant la température entre 30 et 35°C et le pH à 6,5 à l'aide de soude 2N.

A la fin de l'addition, la solution est agitée une heure supplémentaire. Le pH est amené entre 2,5 et 3 par addition d'acide sulfurique concentré (14 ml) afin de précipiter les sels de calcium.

Après filtration, la solution rose a la composition suivante : D-érythrose (87 %), D-arabinose (2 %), acide gluconique (7 %).

Les pourcentages donnés sont ceux correspondants aux résultats analytiques. La somme n'est pas égale à 100 car il y a parfois formation d'un peu d'acide arabinonique (2 %) et d'autres sous-produits.

Ces sous-produits sont : l'acide formique, les "carbonates" et le dioxyde de carbone.

### Exemple 2 :

Le gluconate de calcium monohydrate (115.1g, 0.25 mole), le chlorure de nickel hexahydrate (2.24g, 9.5 mmoles) et de l'eau (1000 ml) sont introduits dans un réacteur double-enveloppe. Le mélange est porté à une température de 40°C et à un pH de 6.5 par addition de soude 2N. L'eau oxygénée à 35 % (95ml, 1.1 moles) est introduite en 70 minutes en maintenant la température entre 40 et 45°C et le pH à 6.5 à l'aide de soude 2N. A la fin de l'addition, la solution est agitée trois heures supplémentaires. Le pH est amené à 2.5 par addition d'acide sulfurique concentré (14ml) afin de précipiter les sels de calcium. Après filtration, la solution verte a la composition suivante : D-érythrose (40 %), acide gluconique (45 %).

Les pourcentages donnés sont ceux correspondants aux résultats analytiques. La somme n'est pas égale à 100 car il y a également formation de glycéraldéhyde (6 %) et d'arabinose (2 %).

Les sous-produits de la réaction sont : l'acide formique, le dioxyde de carbone.

## Revendications

1. Procédé de fabrication de D-érythrose, caractérisé par le fait qu'une solution aqueuse d'un sel de l'acide gluconique est mise au contact de peroxyde d'hydrogène en présence d'un sel d'un métal choisi dans le groupe constitué du cobalt, du nickel et du ruthénium.

2. Procédé de fabrication de D-érythrose selon la revendication 1, caractérisé par le fait que la solution aqueuse a une matière sèche en sel d'acide gluconique comprise entre 1 et 60 %.

3. Procédé de fabrication de D-érythrose selon l'une ou l'autre des revendications 1 et 2, caractérisé par le fait que le sel d'un métal choisi dans le groupe constitué du cobalt, du nickel et du ruthénium est présent en une quantité comprise entre 0,001 et 50 % exprimée par rapport au sel d'acide gluconique.

4. Procédé de fabrication de D-érythrose selon l'une quelconque des revendications 1 à 3, caractérisé par le fait que l'on utilise le peroxyde d'hydrogène, de préférence sous forme d'eau oxygénée d'une richesse de 30 %, en une quantité comprise entre 1 et 500 mol% exprimée par rapport au sel d'acide gluconique.

5. Procédé de fabrication de D-érythrose selon l'une quelconque des revendications 1 à 4, caractérisé par le fait que l'on travaille à une température comprise entre 0 et 100°C et de préférence entre 10 et 50°C.

6. Procédé de fabrication de D-érythrose selon l'une quelconque des revendications 1 à 5, caractérisé par le fait que l'on travaille à un pH compris entre 2 et 12, et de préférence entre 5 et 8.

7. Procédé de fabrication de D-érythrose selon l'une quelconque des revendications 1 à 6, caractérisé par le fait que le sel de l'acide gluconique est obtenu par oxydation du glucose effectuée à l'aide d'air ou d'oxygène, en milieu alcalin, en présence de catalyseurs au palladium.

8. Procédé de fabrication de D-érythrose selon l'une quelconque des revendications 1 à 6, caractérisé par le fait que le sel de l'acide gluconique est obtenu par oxydation du glucose par voie microbiologique.

9. Procédé de fabrication d'érythritol par hydrogénation de D-érythrose, caractérisé par le fait que le D-érythrose est obtenu par le procédé conforme à l'une quelconque des revendications 1 à 8.

## Patentansprüche

1. Verfahren zur Herstellung von D-Erythrose, dadurch gekennzeichnet, dass eine wässrige Lösung eines Salzes der Gluconsäure in Gegenwart eines Salzes eines Metalls, das aus der aus Kobalt, Nickel und Ruthenium bestehenden Gruppe ausgewählt ist, mit Wasserstoffperoxid in Kontakt gebracht wird.

2. Verfahren zur Herstellung von D-Erythrose nach Anspruch 1, dadurch gekennzeichnet, dass die wässrige Lösung einen Gehalt an Trockenmasse von Gluconsäuresalz zwischen 1 und 60 % hat.

3. Verfahren zur Herstellung von D-Erythrose nach einem der Ansprüche 1 und 2, dadurch gekennzeichnet, dass das Salz eines Metalls, das aus der aus Kobalt, Nickel und Ruthenium bestehenden Gruppe ausgewählt ist, in einer Menge zwischen 0,001 und 50 %, bezogen auf das Gluconsäuresalz, vorliegt.

4. Verfahren zur Herstellung von D-Erythrose nach einem der Ansprüche 1 bis 3, dadurch gekennzeichnet, dass man Wasserstoffperoxid vorzugsweise in Form von 30 %igem Wasserstoffperoxid in einer Menge zwischen 1 und 500 Mol%, bezogen auf das Gluconsäuresalz, verwendet.

5. Verfahren zur Herstellung von D-Erythrose nach einem der Ansprüche 1 bis 4, dadurch gekennzeichnet, dass man bei einer Temperatur zwischen 0 und 100 °C und vorzugsweise zwischen 10 und 50 °C arbeitet.

6. Verfahren zur Herstellung von D-Erythrose nach einem der Ansprüche 1 bis 5, dadurch gekennzeichnet, dass man bei einem pH zwischen 2 und 12 und vorzugsweise zwischen 5 und 8 arbeitet.

7. Verfahren zur Herstellung von D-Erythrose nach einem der Ansprüche 1 bis 6, dadurch gekennzeichnet, dass das Salz der Gluconsäure durch Oxidation von Glucose mit Hilfe von Luft oder Sauerstoff in alkalischem Medium in Gegenwart eines Palladiumkatalysators erhalten wird.

8. Verfahren zur Herstellung von D-Erythrose nach einem der Ansprüche 1 bis 6, dadurch gekennzeichnet, dass das Salz der Gluconsäure durch Oxidation von Glucose auf mikrobiologischem Weg erhalten wird.

9. Verfahren zur Herstellung von Erythritol durch Hydrierung von D-Erythrose, dadurch gekennzeichnet, dass die D-Erythrose in dem Verfahren nach einem der Ansprüche 1 bis 8 hergestellt wird.

## Claims

1. Process for the manufacture of D-erythrose, wherein an aqueous solution of a salt of gluconic acid is brought into contact with hydrogen peroxide in the presence of a salt of a metal selected from the group consisting of cobalt, nickel and ruthenium.

2. Process for the manufacture of D-erythrose according to Claim 1, wherein the aqueous solution has a content of salt of gluconic acid, as dry matter, of between 1 and 60%.

3. Process for the manufacture of D-erythrose according to either one of Claims 1 and 2, wherein the salt of a metal selected from the group consisting of cobalt, nickel and ruthenium is present in an amount of between 0.001 and 50%, expressed with respect to the salt of gluconic acid.

4. Process for the manufacture of D-erythrose according to any one of Claims 1 to 3, wherein hydrogen peroxide is used, preferably in the form of an aqueous hydrogen peroxide solution with a concentration of 30%, in an amount of between 1 and 500 mol % expressed with respect to the salt of gluconic acid.

5. Process for the manufacture of D-erythrose according to any one of Claims 1 to 4, wherein the reaction is carried out at a temperature of between 0 and 100 °C, preferably between 10 and 50°C.

6. Process for the manufacture of D-erythrose according to any one of Claims 1 to 5, wherein the reaction is carried out at a pH of between 2 and 12, preferably at a pH of between 5 and 8.

7. Process for the manufacture of D-erythrose, according to any one of Claims 1 to 6, wherein the salt of gluconic acid is obtained by oxidation of glucose carried out using air or oxygen, in alkaline medium, in the presence of palladium catalysts.

8. Process for the manufacture of D-erythrose according to any one of Claims 1 to 6, wherein the salt of gluconic acid is obtained by oxidation of glucose via the microbiological route.

9. Process for the manufacture of erythritol by hydrogenation of D-erythrose, wherein the D-erythrose is obtained by the process in accordance with any one Claims 1 to 8.
